# EUROPÄISCHE PATENTANMELDUNG

(11) **EP 1 233 268 A2**
(43) Veröffentlichungstag der Anmeldung: **21.08.2002**
(21) Anmeldenummer: 02003489.8
(22) Anmeldetag: 15.02.2002
(51) Int. Cl.: G01N 33/487, C12M 3/00

(54) **Vorrichtung zum Durchführen elektrophysiologischer Messungen an Zellen**

(30) Priorität: 16.02.2001 DE 10108968; 16.02.2001 US 785559
(71) Anmelder: Multi Channel Systems MCS GmbH, 72770 Reutlingen (DE)
(72) Erfinder: Boven, Karl-Heinz, 72138 Kirchentellinsfurt (DE); Möller, Andreas, 72072 Tübingen (DE)
(74) Vertreter: Witte, Alexander, Dr.-Ing.

(57) **Zusammenfassung**

Eine Vorrichtung dient zum Durchführen elektrophysiologischer Messungen an Zellen. Sie weist mindestens eine Elektrode (30, 32), eine erste Perfusatleitung, die als Perfusateinlaß (38) mit einer ersten Mündung (39) zum Zuführen von Perfusat zu den Zellen ausgebildet ist, und eine zweite Perfusatleitung auf, die als Perfusatauslaß (40) mit einer zweiten Mündung (41) zum Abführen von Perfusat von den Zellen ausgebildet ist. Die zweite Mündung (41) ist oberhalb der ersten Mündung (39) angeordnet. Die mindestens eine Elektrode (30, 32) ist zum Einstechen in die Zellen ausgebildet und mit den Perfusatleitungen (38, 40) in einem gemeinsamen Träger (22) eines Meßkopfes integriert. Der Perfusateinlaß (38) erstreckt sich im wesentlichen parallel zu der mindestens einen Elektrode (30, 32). Die erste Mündung (39) ist oberhalb eines unteren Endes der mindestens einen Elektrode (30) angeordnet (Fig. 4).

## Beschreibung

Die Erfindung betrifft eine Vorrichtung zum Durchführen elektrophysiologischer Messungen an Zellen, mit mindestens einer Elektrode, mit einer ersten Perfusatleitung, die als Perfusateinlaß mit einer ersten Mündung zum Zuführen von Perfusat zu den Zellen ausgebildet ist, und mit einer zweiten Perfusatleitung, die als Perfusatauslaß mit einer zweiten Mündung zum Abführen von Perfusat von den Zellen ausgebildet ist, wobei die zweite Mündung oberhalb der ersten Mündung angeordnet ist.

Eine Vorrichtung der vorstehend genannten Art ist aus der US 6 048 722 A bekannt.

Die bekannte Vorrichtung dient zum Durchführen elektrophysiologischer Messungen an Oozyten, insbesondere Oozyten des Xenopus laevis, eines südafrikanischen Klauenfrosches. Diese Oozyten werden bevorzugt für elektrophysiologische Messungen als Expressionssystem eingesetzt.

In diesem Zusammenhang ist es bekannt, die Oozyten in einer Aufnahme zu positionieren und zu fixieren. Die Aufnahme kann z.B. eine trichterartige Öffnung in einer Platte sein. -Derartige Platten sind standardisiert und werden üblicherweise mit 8 x 12 = 96, 16 x 24 = 384 oder 32 x 48 = 1.536 derartigen Aufnahmen ("Wells") verwendet. Die Aufnahmen für die Oozyten können z.B. an ihrer Unterseite mit einer Öffnung versehen sein, über die die Oozyte mittels Unterdruck angesaugt und in der Aufnahme fixiert wird.

Zum Durchführen von elektrophysiologischen Messungen wird eine genetische Information, nämlich eine mRNA oder eine cDNA, in die Oozyte eingebracht. Es bilden sich dann an der Oberfläche und in der Oozyte charakteristische Ionenkanäle und/oder Rezeptoren aus, die durch Anlegen einer elektrischen Spannung bzw. Durchleiten eines elektrischen Stroms oder Applikation einer Substanz vermessen werden können.

Es ist bekannt, auf diese Weise pharmakologische Messungen durchzuführen, weil die in der Membran der Oozyten ausgebildeten Rezeptoren oder Ionenkanäle in einer Art und Weise ausgebildet werden, die charakteristisch für bestimmte Eigenschaften der zu untersuchenden Substanzen ist.

Bei der eingangs erwähnten US 6 048 722 werden bei einem ersten Ausführungsbeispiel (Fig. 2) eine Meßelektrode und eine Stimulationselektrode in die Zelle eingestochen, was bedeutet, daß diese Elektroden als Mikroelektroden ausgebildet sind. Die Aufnahme der Zelle ist über einen Rohrbogen mit einem Behälter verbunden, in den eine Referenzelektrode (Masse) eintaucht.

Bei der vorerwähnten Vorrichtung gemäß der US 6 048 722 ist bei einem zweiten Ausführungsbeispiel (Fig. 3), das der eingangs genannten Vorrichtung entspricht, die Zufuhr von Perfusat automatisiert. Bei diesem Ausführungsbeispiel ist die Zelle am Boden eines abgeknickten Röhrchens angeordnet, wobei das Röhrchen an dieser Stelle eine Öffnung aufweist, durch die die Zelle sich teilweise vorstülpen kann. Mittels eines weiteren, sehr dünnen Röhrchens kann die Zelle an dieser vorgestülpten Stelle durchstochen werden, um eine elektrische Verbindung zum Zellinneren herzustellen. Der Boden des abgeknickten Röhrchens befindet sich in einem gefäßartigen Gebilde, das durch einen um den Boden herumgewickelten Streifen gebildet wird. In diesem Gefäß befinden sich zwei Meßelektroden, die jedoch nicht als Mikroelektroden ausgebildet und nicht in die Zelle eingestochen sind. Sie stehen lediglich elektrisch mit dem Innenraum der Zelle in Verbindung.

An die Aufnahme der Zelle ist eine Perfusionseinrichtung angeschlossen. Mit dieser Perfusionseinrichtung lassen sich in gesteuerter Weise unterschiedliche Substanzen, insbesondere Meßsubstanzen, in die Aufnahme einfüllen, die typischerweise ein Fassungsvermögen von 100 µl hat. Im abgeknickten Bereich des Röhrchens ist horizontal neben der Rückseite der Zelle ein freies Ende eines Schlauchs angeordnet, der als Perfusateinlaß dient. In einem schräg nach oben weisenden Bereich des abgeknickten Röhrchens befindet sich das freie Ende eines zweiten Schlauchs, der als Perfusatauslaß verwendet wird. In deren unmittelbarer Nähe befindet sich in dem abgeknickten Röhrchen noch eine Referenzelektrode.

Auch für dieses zweite Ausführungsbeispiel gemäß der US 6 048 722 gilt daher, daß es sich um einen experimentellen Aufbau handelt, der jeweils einzeln aufgebaut und justiert werden muß.

Allgemein ist somit bei bekannten Vorrichtungen der hier interessierenden Art ein erhebliches handwerkliches Geschick erforderlich, um die Perfusatleitung in den Bereich der Aufnahme für die Zelle zu bringen und dort zu fixieren. Vor allem aber ist auch die Anbringung der Elektroden an der Zelle, insbesondere das Einstechen der Elektroden in die Zelle, von der Geschicklichkeit der jeweiligen Untersuchungsperson abhängig, da dies offensichtlich von Hand geschieht. Sofern die Applikation der Elektrode bzw. der Elektroden an die Zelle mißlingt, insbesondere wenn die Elektroden abbrechen, muß der gesamte Meßaufbau neu eingerichtet und neu justiert werden. Schließlich ist mit der bekannten Vorrichtung nur jeweils eine Einzelmessung an einer Zelle möglich, wobei lediglich die Abfolge unterschiedlicher Testflüssigkeiten in der erwähnten Weise automatisch gesteuert werden kann.

Aus der US 4 461 304 sind eine Mikroelektrode sowie eine Anordnung zum parallelen Messen neurologischer Zellen bekannt. Bei dieser bekannten Anordnung ist eine Elektrode vorgesehen, die an ihrem unteren freien Ende mit einem auswechselbaren, nadelartigen Element versehen ist. In makroskopischer Ansicht ist das nadelartige Element flach und sich nach vorne verjüngend ausgebildet. Auf der Oberfläche des Elementes befinden sich im axialen Abstand aufgereiht insgesamt 20 Elektrodenpunkte, die jeweils über eine separate gedruckte Leiterbahn angeschlossen sind. Diese Leiterbahnen führen über entsprechende Kontakt- und Verbindungselemente zu einer oberhalb des nadelartigen Elementes im Elektrodenträger angeordneten Schaltleiste.

Diese bekannte Vorrichtung ist daher lediglich für spezielle Messungen geeignet, bei denen viele (beispielsweise 20) nebeneinander liegende Meßpunkte in neurologischem Gewebe vermessen werden sollen.

Der Erfindung liegt demgegenüber die Aufgabe zugrunde, eine Vorrichtung der eingangs genannten Art dahingehend weiterzubilden, daß die vorstehend erläuterten Nachteile vermieden werden.

Insbesondere soll es möglich sein, die Messung sowohl hinsichtlich der Applikation der Elektroden wie auch hinsichtlich der Applikation der Perfusatleitungen vollautomatisch durchzuführen, weiterhin soll die Vorrichtung nach dem "plug-and-play"-Prinzip mit wenigen Handgriffen einsetzbar und bei einer eventuellen Beschädigung wieder-einsetzbar gemacht werden können. Schließlich soll es möglich werden, eine Vielzahl von Messungen an vielen unterschiedlichen Zellen vollautomatisiert, d.h. ohne Aufsicht durchführen zu können, insbesondere über Nacht.

Diese Aufgabe wird gemäß der eingangs genannten Vorrichtung erfindungsgemäß dadurch gelöst, daß die mindestens eine Elektrode zum Einstechen in die Zellen ausgebildet und mit den Perfusatleitungen in einem gemeinsamen Träger eines Meßkopfes integriert ist, daß der Perfusateinlaß sich im wesentlichen parallel zu der mindestens einen Elektrode erstreckt, und daß die erste Mündung oberhalb eines unteren Endes der mindestens einen Elektrode angeordnet ist.

Die Erfindung hat den Vorteil, daß der Träger industriell vorgefertigt werden kann und lediglich in der Vorrichtung an eine entsprechende Halterung eingesteckt werden muß. Die Elektroden sind dabei fertig installiert, insbesondere hinsichtlich ihrer relativen Lage zueinander, so daß das bei herkömmlichen Vorrichtungen notwendige und sehr delikate Ausrichten der Elektroden zueinander nicht mehr erforderlich ist. Auf diese Weise wird erreicht, daß das Risiko einer Beschädigung der Elektroden beim Einrichten der Vorrichtung drastisch minimiert wird. Außerdem werden die Messungen deutlich reproduzierbar, weil die Elektroden sich in einer definierten Lage zueinander befinden. Schließlich gestattet die integrierte Anordnung der Elektroden in dem Träger, daß automatisierte Verfahreinrichtungen für den so gebildeten Meßkopf gebildet werden, so daß die gewünschten vollautomatisierten Messungen an einer Vielzahl von Zellen möglich werden, beispielsweise in Verbindung mit einer standardisierten Multi-Well-Platte.

Die Maßnahme, auf dem Träger ferner mindestens eine Perfusatleitung anzuordnen, hat den Vorteil, daß die Perfusionseinrichtung hinsichtlich der Zuführung und der Abführung des Perfusats in dem selben Meßkopf integriert ist, in dem sich bereits Elektroden befinden, so daß eine gemeinsame Handhabung möglich ist. Weiterhin ist von Vorteil, daß beim Integrieren auch der Perfusatleitungen in dem Meßkopf die relative Positionierung der Perfusatleitungen zu den Elektroden optimiert werden kann und bereits herstellerseitig fixiert ist. Auch insoweit entfällt ein umständliches Handhaben der Vorrichtung beim Aufbau der Meßanordnung. Weiterhin ergibt sich der Vorteil, daß bei einer Beschädigung einer der Komponenten der gesamte Meßkopf mit wenigen Handgriffen ausgetauscht werden kann.

Da der Perfusateinlaß eine erste Mündung aufweist, der Perfusateinlaß ferner im wesentlichen parallel zu der mindestens einen Elektrode angeordnet ist und schließlich die erste Mündung oberhalb eines unteren Endes der mindestens einen Meßelektrode angeordnet ist, wird das Perfusat ganz gezielt an exakt die Stelle geleitet, an der sich der aktive Teil der Meßelektroden befindet.

Das Vorsehen eine Perfusatauslasses mit einer zweiten Mündung hat den Vorteil, daß das nicht mehr benötigte Perfusat kontrolliert abgeführt und insbesondere ein Überlaufen der Aufnahme für die Zelle verhindert werden kann. Da die zweite Mündung oberhalb der ersten Mündung angeordnet ist, kann die Zelle über die Strecke zwischen den beiden Mündungen stets mit frischem Perfusat versorgt werden, d.h. entweder mit einer Testflüssigkeit oder zwischen den Meßvorgängen mit einer Spülflüssigkeit.

Diese Maßnahme hat daher den weiteren Vorteil, daß auch außerhalb von eigentlichen Meßvorgängen ein gezieltes Zuführen von Perfusat möglich ist, um die gerade nicht vermessenen Zellen gegen Austrocknen zu schützen. Dann kann über den vertikalen Abstand der beiden Mündungen in genau definierter Weise ein vorgegebener Flüssigkeitsspiegel oberhalb der Zellen eingestellt werden.

Bei bevorzugten Ausführungsformen der erfindungsgemäßen Vorrichtung sind die Elektroden in Aussparungen des Trägers eingesetzt, sie können aber auch im Träger vergossen sein.

Diese Maßnahmen haben den Vorteil, daß eine stabile und reproduzierbare Lage der Elektroden im Träger erreicht wird.

Bei weiteren Ausführungsformen der Erfindung bestehen die Elektroden aus gezogenen Glasröhrchen.

Diese Maßnahme hat den Vorteil, daß derartige Glaselektroden in an sich bekannter Weise für den jeweiligen Anwendungszweck optimiert ausgebildet werden können. So ist es z.B. möglich, die Elektroden mit einem elektrischen Widerstand zwischen 5 MΩ und 100 MΩ auszubilden, und zwar als sogenannte "sharp electrodes". Alternativ können die Elektroden auch als sogenannte "patch electrodes" mit einem elektrischen Widerstand in der Größenordnung von 500 kΩ bis 5 MΩ ausgebildet werden.

Alternativ ist erfindungsgemäß aber auch vorgesehen, Elektroden als Drahtelektroden auszubilden, vorzugsweise als Silberdrahtelektroden, die weiter vorzugsweise mit einer Chloridschicht umgeben sind. Daneben ist auch die Verwendung von Wolframdrähten und dergleichen möglich.

Bei bevorzugten Ausführungsformen der erfindungsgemäßen Vorrichtung weist mindestens eine Elektrode einen geraden Abschnitt auf.

Diese Maßnahme hat den Vorteil, daß die Elektrode in besonders einfacher Weise im Träger befestigt werden kann.

Wenn in weiter bevorzugter Ausbildung der Erfindung zwei Elektroden im wesentlichen symmetrisch zu einer Längsachse des Trägers angeordnet sind, so kann man den Abstand zwischen den freien Enden der Elektroden in einem Bereich zwischen etwa 50 µm und 800 µm, vorzugsweise zwischen 200 µm und 500 µm einstellen.

Diese Maßnahme hat den Vorteil, daß beim Einführen der Elektroden in die Zellen individuelle Einstechlöcher erzielt werden und nicht ein gemeinsames, großes Loch aufgerissen wird, das entstünde, wenn die freien Enden der Elektroden zu dicht beieinander angeordnet wären. Dies hätte nämlich den Nachteil, daß wichtige physiologische Funktionen der Zelle verlorengingen. Dies wird bei der erfindungsgemäßen Vorgehensweise vermieden.

In diesem Zusammenhang ist besonders bevorzugt, wenn ein gerader Abschnitt der Elektroden mit einer Längsachse des Trägers einen spitzen Winkel einschließt, der insbesondere zwischen 3° und 10°, vorzugsweise 5° betragen kann.

Diese zueinander geneigte Anordnung der Elektroden hat den Vorteil, daß die unteren freien Enden der Elektroden in gut reproduzierbarer Weise positioniert werden können.

Bei einer bevorzugten Gruppe von Ausführungsbeispielen der Erfindung ist mindestens eine der Elektroden als Meßelektrode ausgebildet. Die Meßelektrode ist vorzugsweise an einen Meßverstärker angeschlossen, der weiter vorzugsweise einstellbar ist.

Auf diese Weise ist es möglich, automatisch gesteuerte Messungen von Spannungs- oder Stromsignalen durchzuführen.

Dies gilt insbesondere dann, wenn die mindestens eine Meßelektrode an eine Stromquelle angeschlossen ist und diese wiederum vorzugsweise einstellbar ist.

Diese Maßnahme hat den Vorteil, daß bei Einsatz zweier unterschiedlicher Elektroden eine Entkopplung zwischen der Stromeinleitung einerseits und der Spannungsmessung andererseits erreicht wird.

Die hier zu messenden Ströme und Spannungen liegen im nA- bis µA-Bereich für die Strommessung, und im mV-Bereich für die Spannungsmessung.

Diese Messungen können durch die gängigen elektrophysiologischen Meßmethoden wie "bridge-mode", "current-clamp", und "voltage-clamp" durchgeführt werden. Dabei ist es für diese Erfindung unerheblich, ob die voltage-clamp-Ableitung mit zwei Elektroden (two-electrode voltage clamp; TEVC) oder mit einer Elektrode (single-electrode voltage-clamp, SEVC) durchgeführt wird. Bei der SEVC-Methode wird im sogenannten "switched-mode" gemessen, also abwechselnd wird in einem bestimmten Intervall ein Meßstrom eingeleitet und die Meßspannung (bei abgeschalteter Strominjektion) gemessen.

Zu diesem Zweck ist bei weiteren Ausführungsbeispielen bevorzugt, wenn mindestens eine der Elektroden als Referenzelektrode ausgebildet ist. In diesem Fall ist vorzugsweise die Referenzelektrode an eine Masse angeschlossen.

Zu dem erwähnten Zweck sind Konfigurationen besonders bevorzugt, bei denen zwei Meßelektroden und zwei Referenzelektroden vorgesehen sind.

In diesem Falle ist besonders bevorzugt, wenn mindestens zwei Meßelektroden in einer ersten gemeinsamen Ebene und/oder mindestens zwei Referenzelektroden in einer zweiten gemeinsamen Ebene angeordnet sind und weiter vorzugsweise die erste und die zweite Ebene parallel zueinander verlaufen und einen möglichst geringen Abstand zueinander aufweisen.

Diese Maßnahmen haben den Vorteil, daß ein extrem kompakter und von der Meßtechnik her optimaler Aufbau entsteht, bei dem alle erforderlichen Komponenten auf engstem Raum vereinigt sind.

Dies gilt insbesondere dann, wenn der Perfusateinlaß bei der bereits weiter oben erwähnten symmetrischen Anordnung der Elektroden im wesentlichen auf der Symmetrieachse zwischen den Meßelektroden angeordnet ist.

Es ist weiterhin in diesem Zusammenhang bevorzugt, wenn der Perfusateinlaß an eine Förderpumpe angeschlossen und diese insbesondere einstellbar ist.

Diese Maßnahme hat den Vorteil, daß das Perfusat in genau dosierter Weise zugeführt werden kann.

Bei weiteren Varianten dieses Ausführungsbeispiels ist der Perfusateinlaß über ein steuerbares Ventilsystem an eine Mehrzahl von Vorratsbehältern anschließbar, die eine Testflüssigkeit oder eine Spülflüssigkeit enthalten können.

Diese Maßnahme hat den Vorteil, daß hinsichtlich der Perfusionseinrichtung vollautomatisierte Messungen mit den bereits erwähnten Vorteilen durchgeführt werden können.

Besonders ist bevorzugt, wenn die Vorratsbehälter oberhalb des Perfusateinlasses angeordnet sind.

Dann ist es nämlich möglich, auf die Förderpumpe zu verzichten, weil dann nach Öffnen des Ventils in der entsprechenden Verbindungsleitung die Test- oder Spülflüssigkeit von selbst unter Schwerkrafteinfluß zum Perfusateinlaß strömt.

Eine besonders gute Wirkung wird erzielt, wenn die Mündungen der Perfusatleitungen entgegengesetzt gerichtet sind.

Diese Maßnahme hat den Vorteil, daß ein Kurzschluß zwischen dem Einlaß- und dem Auslaßsystem verhindert wird, wenn in entgegengesetzten Richtungen eingelassen bzw. abgesaugt wird.

Auch in diesem Falle ist sinngemäß bevorzugt, wenn der Perfusatauslaß an eine Saugpumpe angeschlossen und diese insbesondere einstellbar ist.

Eine besonders gute Wirkung wird erzielt, wenn in Blickrichtung auf die erste Ebene der Perfusateinlaß vor der ersten Ebene und der Perfusatauslaß hinter der zweiten Ebene angeordnet ist.

Diese Maßnahme hat den Vorteil, daß ein extrem kompakter und sicherer Aufbau entsteht, bei dem alle für die Messung erforderlichen Komponenten in optimaler Weise zusammenarbeiten.

Bei Ausführungsbeispielen der Erfindung ist bevorzugt, wenn mindestens ein Meßkopf an einem Aktuator angeordnet ist und der Aktuator entlang eines Koordinatensystems oberhalb einer Aufnahme für die Zellen verfahrbar ist.

Diese Maßnahme hat den Vorteil, daß der Meßkopf in allen Richtungen des Koordinatensystems vollautomatisch verfahren werden kann, so daß alle notwendigen Bewegungen programmiert durchgeführt werden können. Dies betrifft insbesondere das Anstechen der Zellen mittels der Meßelektroden, aber auch das Heranfahren des Meßkopfes an die Zellen, wenn lediglich eine Befeuchtung der Zellen mittels Perfusat gewünscht ist, wie dies vorstehend erläutert wurde.

In einer bevorzugten Weiterbildung dieses Ausführungsbeispiels kann der Aktuator eine Mehrzahl von Meßköpfen tragen.

Diese Maßnahme hat insbesondere bei der Verwendung von sogenannten Multi-Well-Platten den Vorteil, daß mehrere Zellen entlang einer Reihe oder einer Spalte der Platte parallel und gleichzeitig gemessen werden können, so daß die in den Wells der Platte enthaltenen Zellen insgesamt in einem Bruchteil der ansonsten benötigten Zeit gemessen werden können. Dabei kann selbstverständlich innerhalb der verschiedenen Aufnahmen für die Zellen mit unterschiedlichen Perfusaten gearbeitet werden, d.h. mit unterschiedlichen Testflüssigkeiten oder mit Spülflüssigkeiten. Wenn unterschiedliche Testflüssigkeiten verwendet werden, kann dies bedeuten, daß an sich dieselbe Art Testflüssigkeit, jedoch in unterschiedlicher Konzentration, verwendet wird, oder aber es können Testflüssigkeiten ganz unterschiedlicher Art eingesetzt werden.

Zu diesem Zweck ist es zweckmäßig, wenn die Meßköpfe relativ zum Aktuator mindestens entlang der auf die Zelle gerichteten Achse individuell verfahrbar sind.

Diese Maßnahme hat den Vorteil, daß die Bewegungsvorgänge an einer Zelle individuell eingestellt werden können, auch wenn in der geschilderten Weise mehrere Zellen parallel vermessen werden.

Bei einer besonders bevorzugten Ausbildung der erfindungsgemäßen Vorrichtung ist der Meßkopf steckbar oder schraubbar am Aktuator befestigt.

Diese Maßnahme hat den Vorteil, daß ein schneller Austausch des Meßkopfs möglich ist, ohne daß der gesamte Versuchsaufbau verändert werden muß.

In weiterer bevorzugter Ausgestaltung der Erfindung sind Mittel zum Injizieren von cDNA und/oder mRNA in die Zelle vorgesehen. Dies geschieht vorzugsweise dadurch, daß diese Mittel am Aktuator angeordnet sind.

Diese Maßnahmen haben den Vorteil, daß auch während des an sich bekannten Schritts des Injizierens dies in der erwähnten Weise automatisiert und gegebenenfalls für eine Vielzahl von Zellen gesteuert nacheinander stattfinden kann.

Es wurde bereits erwähnt, daß die Erfindung in besonders vorteilhafter Weise dann eingesetzt werden kann, wenn die Aufnahme für die Zellen als standardisierte Multi-Well-Platte ausgebildet ist.

In diesem Fall wird eine besonders gute Wirkung dann erzielt, wenn die einzelnen Aufnahmen in der Platte mit einer lesbaren Codierung versehen sind und der Aktuator Mittel zum Lesen der Codierung umfaßt. Dies gilt vor allem dann, wenn die Codierung eine Bar-Codierung ist und die Mittel ein Bar-Code-Lesekopf sind.

Diese Maßnahme hat den Vorteil, daß die erfindungsgemäße Vorrichtung zu Beginn des automatisierten Meßvorganges für viele Zellen an einer bestimmten, vorbestimmten Zelle in einem vorbestimmten Well beginnen kann und dann je nach Programmierung ihren Weg über die Mulit-Well-Platte fortsetzt. An jeder einzelnen Aufnahme, insbesondere an jedem einzelnen Well, kann dann durch Lesen des Bar-Codes überprüft werden, ob die Position des Meßkopfes insoweit korrekt ist.

Weitere Vorteile ergeben sich aus der Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine Draufsicht auf einen Teil einer Multi-Well-Platte, wie sie vorteilhaft im Rahmen der vorliegenden Erfindung verwendet werden kann;
- Fig. 2: eine Seitenansicht, teilweise im Schnitt, entlang der Linie II-II von Fig. 1, durch ein Ausführungsbeispiel einer erfindungsgemäßen Vorrichtung;
- Fig. 3: in noch weiter vergrößertem Maßstab eine Vorderansicht eines Meßkopfes, wie er in der Vorrichtung gemäß Fig. 2 verwendet werden kann;
- Fig. 4: eine Seitenansicht, teilweise im Schnitt, durch den Meßkopf gemäß Fig. 3;
- Fig. 5: in noch weiter vergrößertem Maßstab eine Schnittdarstellung entlang der Linie V-V von Fig. 4 zur Erläuterung weiterer Einzelheiten des dort dargestellten Meßkopfes; und
- Fig. 6: einen Aufschrieb einer Messung, wie sie mit der Vorrichtung gemäß Fig. 2 bis 5 durchführbar ist.

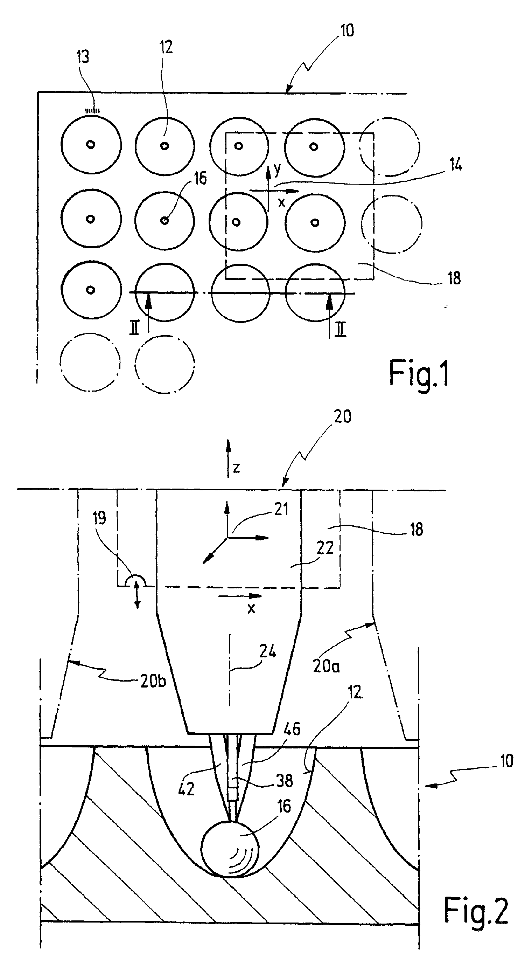

In Fig. 1 bezeichnet 10 insgesamt eine sogenannte Well-Platte, wie sie bei elektrophysiologischen Messungen an Oozyten verwendet wird. Well-Platten 10 sind standardisiert und enthalten eine Vielzahl von Wells 12, d.h. Aufnahmen für Oozyten. Standardisierte Well-Platten 10 sind mit Wells 12 in Reihen und Spalten versehen, wobei Formate von 8 x 12 = 96, 16 x 24 = 384 oder 32 x 48 = 1.536 Wells üblich sind.

Zum Auffinden und Identifizieren einzelner Wells 10 sind Codierungen vorgesehen, von denen in Fig. 1 eine bei 13 als Bar-Code angedeutet ist.

Die Reihen und Spalten der Wells 12 in der Well-Platte 10 definieren ein Koordinatensystem 14, bei standardisierten Well-Platten 10 ist dies ein kartesisches Koordinatensystem mit Achsen x und y.

In Fig. 1 ist in den Wells 12 bei 16 jeweils eine Oozyte angedeutet. Bei elektrophysiologischen Messungen der hier interessierenden Art werden üblicherweise Oozyten des südafrikanischen Klauenfrosches (Xenopus laevis) verwendet. Diese Oozyten 16 haben einen Durchmesser von ca. 1 mm. Die Wells 12 sind, wie auch aus der Schnittdarstellung gemäß Fig. 2 ersichtlich ist, von gewölbter Gestalt, die sich vorzugsweise nach unten hin verjüngt.

Um die Oozyten 16 in den Wells 12 zu fixieren, kann vorgesehen sein, die Wells 12 am tiefsten Punkt mit einem Kanal an ein Unterdrucksystem anzuschließen (in den Figuren nicht dargestellt), um die Oozyten 16 in den Wells 12 mittels Unterdruck zu fixieren.

In Fig. 1 und 2 ist mit 18 ein Aktuator angedeutet, der entlang der Ebenenkoordinaten x und y, aber auch entlang einer dazu senkrechten Koordinate z verfahrbar ist. Zu diesem Zweck ist der Aktuator 18 an ein elektronisches Steuergerät (nicht dargestellt) angeschlossen, das entsprechenden Bewegungseinheiten für die drei Koordinaten x, y und z die erforderlichen Steuerbefehle zuleitet. Diese dreidimensionale Bewegungsmöglichkeit des Aktuators 18 ist in Fig. 2 bei 21 nochmals angedeutet.

Der Aktuator 18 weist an seiner Unterseite einen Lesekopf 19 auf. Der Lesekopf 19 ist z.B. als Bar-Code-Leser ausgebildet. Er ist imstande, die Codierung 13 an jedem Well 12 zu identifizieren, so daß jedes Well 12 in seinen Koordinaten auf der Well-Platte 10 identifiziert werden kann.

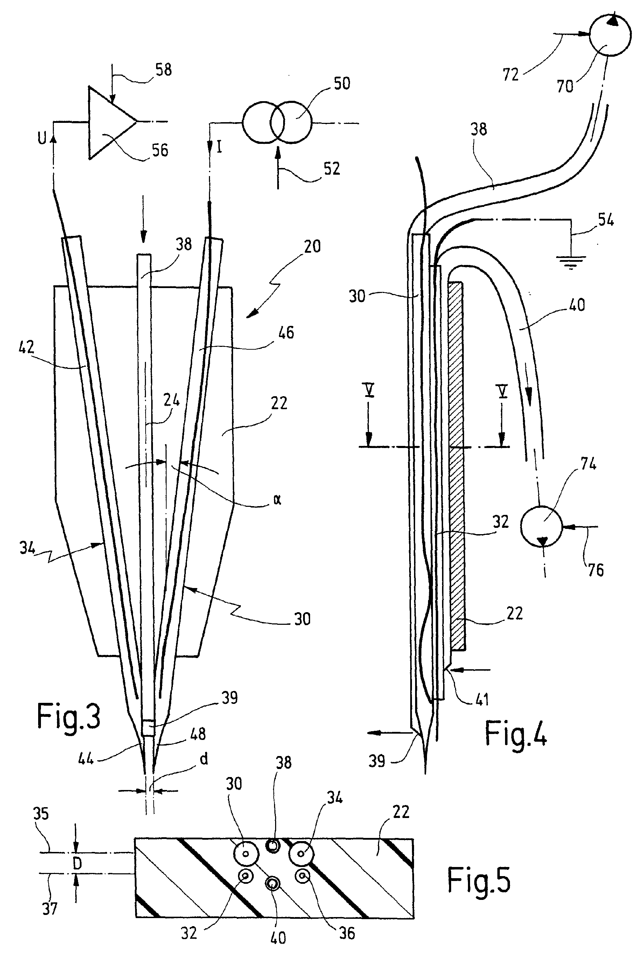

Der Aktuator 18 trägt einen Meßkopf 20, mit dem in noch zu beschreibender Weise die Messungen an den Oozyten 16 durchgeführt werden. Der Meßkopf 20 weist einen Träger 22 auf, in den die dazu erforderlichen Elemente integriert sind, wie weiter unten anhand der Fig. 3 bis 5 noch im einzelnen erläutert werden wird. Der Träger 22 hat eine Längsachse 24, die im dargestellten Ausführungsbeispiel mit der vertikalen z-Achse zusammenfällt.

In Fig. 2 ist mit 20a und 20b noch angedeutet, daß oberhalb der Well-Platte 10 nicht nur ein Meßkopf 20 sondern auch mehrere Meßköpfe 20, 20a, 20b ... angeordnet sein können. Vorzugsweise sind die Meßköpfe 20, 20a, 20b in einer Reihe angeordnet, so daß gleichzeitig eine Mehrzahl von Oozyten 16 in ihren Wells 12 vermessen werden können. Dies kann beispielsweise eine komplette Reihe oder Spalte von Wells 12 in der Well-Platte betreffen.

Vorzugsweise wird dabei so vorgegangen, daß zwar die gesamte Reihe von Meßköpfen 20, 20a, 20b gemeinsam über die Well-Platte 10 verfahren wird, um nacheinander verschiedene Reihen bzw. Spalten von Wells 12 anzufahren. Bevorzugt bleibt dabei jedoch, daß zumindest der z-Antrieb jedes Aktuators 18 für jeden einzelnen Meßkopf 20, 20a, 20b ... indivuduell steuerbar bleibt, damit nach dem Anfahren der Wells 12 die eigentliche Messung an jeder Oozyte 16 individuell durchgeführt werden kann.

In den Fig. 3 bis 5 ist der Meßkopf 20 in weiteren Einzelheiten dargestellt.

Jeder Meßkopf 20 weist dabei eine erste Elektrode 30, eine erste Referenzelektrode 32, eine zweite Elektrode 34 sowie eine zweite Referenzelektrode 36 auf.

Wie man aus der vergrößerten Querschnittsdarstellung gemäß Fig. 5 erkennen kann, liegen die beiden Elektroden 30 und 34 in einer gemeinsamen ersten Ebene 35, und die beiden Referenzelektroden 32 und 36 in einer zweiten Ebene 37. Die beiden Ebenen 35 und 37 erstrecken sich parallel zueinander und haben voneinander einen Abstand D.

In dem Träger 22 befindet sich ferner ein Perfusionseinsatz 38 mit einer unteren Mündung 39 sowie ein Perfusatauslaß 40 mit einer unteren Mündung 41. In Blickrichtung auf die erste Ebene 35 liegt der Perfusateinlaß 38 vor der ersten Ebene 35 und der Perfusatauslaß 40 hinter der zweiten Ebene 37. Der Perfusateinlaß 38 und der Perfusatauslaß 40 liegen in der Mittelebene zwischen den Elektroden 30, 34 und den Referenzelektroden 32 und 36. Diese fällt mit der Längsachse 24 des Trägers 22 zusammen.

Die Elektroden 30 und 34 weisen jeweils einen geraden Abschnitt 42 bzw. 46 auf und laufen unten in eine Spitze 44 bzw. 48 aus. Die geraden Abschnitte 42, 46 sind zur Längsachse 24 des Trägers 22 geneigt, und zwar unter einem Winkel α, der vorzugsweise zwischen 3° und 10°, insbesondere 5° beträgt.

Die Anordnung ist dabei so gewählt, daß die Spitzen 44 und 48 einen sehr geringen Abstand d voneinander haben, der zwischen 200 µm und 500 µm beträgt. Demgegenüber liegt der Abstand d zwischen den Ebenen 35 und 37 in der Größenordnung von 1 mm.

Die Referenzelektroden 32 und 36 liegen in Fig. 3 hinter den Meßelektroden 30, 34 und sind in gleicher Weise geneigt angeordnet.

Die Elektroden 30 und 34 dienen, wie erwähnt, als Meß- bzw. Ableitelektroden. Sie können als sogenannte Glaselektroden aus gezogenen Glasröhrchen bestehen. Diese werden im elektrophysiologischen Sprachgebrauch als "sharp electrodes" bezeichnet, wenn sie einen elektrischen Widerstand in der Größenordnung von 5 bis 100 MΩ aufweisen oder als "patch electrodes", wenn ihr elektrischer Widerstand in der Größenordnung von 500 kΩ bis 5 MΩ liegt. Die sogenannten "patch electrodes" werden in der Praxis sowohl zum Einstecken wie auch zum Ansaugen verwendet. Alternativ können aber auch Drahtelektroden verwendet werden, die z.B. aus Wolfram bestehen und die als "wire electrodes" bezeichnet werden.

Die Referenzelektroden 32 und 36 sind hingegen vorzugsweise als Drahtelektroden ausgebildet. Zu diesem Zweck können Silberdrähte verwendet werden, die mittels eines elektrolytischen Prozesses mit einer Chloridschicht versehen sind. Derartige Elektroden werden als Ag/AgCl-Elektroden bezeichnet.

Die Perfusatleitungen 38 und 40 sind als Schläuche ausgebildet.

Die sechs vorgenannten Elemente 30, 32, 34, 36, 38 und 40 sind in der in Fig. 5 dargestellten Weise in den Träger 22 des Meßkopfes 20 integriert. Unter "integriert" können dabei verschiedene Anbringungsarten verstanden werden. So können die Elemente in entsprechende Bohrungen, Ausnehmungen, Ausfräsungen und dergleichen innerhalb eines einstückigen Trägerkörpers eingelegt, eingesteckt oder sonstwie fixiert sein. Es ist aber auch möglich, die genannten Elemente gesamthaft in einen Träger 22 einzugießen. Wichtig ist allein, daß alle genannten Elemente, d.h. alle Elektroden und Perfusatleitungen, nach Lage und Abmessungen im Träger 22 fixiert sind, so daß sie als "plug-and-play"-Einheit leicht montiert und gegebenenfalls ausgetauscht werden können. Hierzu ist der Träger 22 vorzugsweise an den Meßkopf 20 ansteckbar oder sonstwie in lösbarer Weise zu befestigen.

In Fig. 3 und 4 ist noch die zugehörige Verschaltung der vorerwähnten Elemente angedeutet.

So erkennt man, daß die erste Meßelektrode 30 an eine Stromquelle 50 angeschlossen ist, die über einen Steueranschluß 52 einstellbar ist. Die Stromquelle 50 liefert einen Strom I an die erste Meßelektrode 30.

Die zweite Meßelektrode 34 ist hingegen an einen Meßverstärker 56 angeschlossen, der einen Steuereingang 58 aufweist. Im Meßverstärker 56 wird das von der zweitem Meßelektrode 34 erfaßte Spannungssignal U verstärkt und/oder sonstwie weiterverarbeitet.

Der Perfusateinlaß 38 ist mit einer Förderpumpe 70 verbunden, die ihrerseits einen Steuereingang 72 aufweist. Die Förderpumpe 70 kann über ein entsprechendes Ventilsystem an eine Vielzahl von Vorratsbehältern angeschlossen sein, in denen sich verschiedenartige Meß- und Spülsubstanzen befinden.

Der Perfusatauslaß 40 ist demgegenüber an eine Saugpumpe 74 angeschlossen, die einen Steuereingang 76 aufweist.

Die erfindungsgemäße Vorrichtung ist ferner mit Elementen versehen, die es gestatten, die zu messenden Oozyten 16 mit einer cDNA und/oder einer mRNA zu impfen. Die hierzu erforderlichen Bauelemente sind vorzugsweise ebenfalls mit dem Aktuator 18 gekoppelt, um auch diese zu jeder einzelnen Oozyte 16 in jedem vorgewählten Well 12 fahren zu können.

Mit Hilfe der vorstehend erläuterten Vorrichtung können folgende Messungen durchgeführt werden:

Zunächst werden in die beispielsweise 96 Wells 12 der Well-Platte 10 Oozyten 16 eingebracht. Die Oozyten 16 werden dann sequentiell oder zu Gruppen gleichzeitig mit cDNA oder mRNA versehen, indem diese Substanzen in die Oozyten 16 injiziert werden. Die Oozyten werden dann inkubiert, z.B. über zwei oder mehr Tage hinweg.

Für die sich nun anschließende Messung wird der Aktuator 18 zunächst entlang des Koordinatensystems 14, d.h. in x- und y-Richtung oberhalb der Well-Platte 10 verfahren, bis er ein vorgewähltes Well 12 erreicht hat, was durch Lesen des entsprechenden Bar-Codes 13 erkannt wird.

In dieser Position wird der Meßkopf 20 durch Verfahren der z-Achse abgesenkt, bis sich die Spitzen 44, 48 oberhalb der Oozyte 16 befinden.

Die Anordnung arbeitet nun zunächst im "current clamp"-Betrieb, in dem der Strom im nA- bis µA-Bereich konstant gehalten wird. Dies geschieht durch Einstellen der Stromquelle 50 über den Steuereingang 52. Die Stromquelle 50 ist hier als Idealstromquelle angenommen, so daß der einmal eingestellte Strom I selbsttätig konstant gehalten wird.

Infolge des zu diesem Zeitpunkt relativ niedrigen Widerstandes stellt sich an der Meßelektrode 34 eine relativ geringe Meßspannung U ein, die im Verstärker 56 erfaßt wird.

Der Meßkopf 20 wird nun weiter in z-Richtung abgesenkt, bis die Spitzen 44, 48 in die Oozyte 16 eindringen. Dabei entstehen zwei um den Abstand d beabstandete Löcher in der Oozyte 16.

Aufgrund des sich schlagartig vergrößernden Widerstandes tritt nun ein erheblicher Spannungssprung in der Meßspannung U auf.

Die Anordnung schaltet nun vom "current clamp"-Betrieb in den "voltage clamp"-Betrieb um. In dieser Betriebsweise wird die Spannung U im Meßverstärker 56 dadurch konstant gehalten, daß der Strom I über die steuerbare Stromquelle 50 jeweils entsprechend nachgeregelt wird.

In diesem Augenblick ist die Förderpumpe 70 an ein Vorratsgefäß für eine Testsubstanz angeschlossen, oder die Testsubstanz steht unter Schwerkraft von selbst am Perfusateinlaß 38 an. Die Förderpumpe 70 wird nun über den Steuereingang 72 eingeschaltet (und/oder ein entsprechendes Ventil in der Zuleitung zum Perfusateinlaß 38 geöffnet), so daß eine Testsubstanz einströmen kann, wie in Fig. 4 mit Pfeilen angedeutet. Die Testsubstanz fließt durch den Perfusateinlaß 38 und unten aus der Mündung 39 hinaus. Die Mündung 39 ist dabei so gerichtet, daß die Testsubstanz waagerecht in Fig. 4 nach links abströmt. Gleichzeitig wird die Saugpumpe 74 eingeschaltet, so daß überschüssige Testsubstanz über die Öffnung 41 und den Perfusatauslaß 40 abgesaugt werden kann. Auch die Öffnung 41 ist waagerecht gerichtet, jedoch zur Öffnung 39 entgegengesetzt. Dies bewirkt, daß ein Kurzschluß zwischen den Öffnungen 39 und 41 weitgehend vermieden wird.

Insgesamt wird auf diese Weise erreicht, daß die Testsubstanz die Oozyte 16 mit einem vorwählbaren Niveau überdeckt, das durch den vertikalen Abstand der Öffnungen 39 und 41 zueinander bestimmt ist.

Durch das Aufbringen der Testsubstanz verändert sich die Oozyte 16, indem Ionenkanäle geöffnet werden.

Aufgrund dessen treten Stromänderungen im nA- bis µA-Bereich auf, die über den Meßverstärker 56 gemessen und anschließend aufgezeichnet werden.

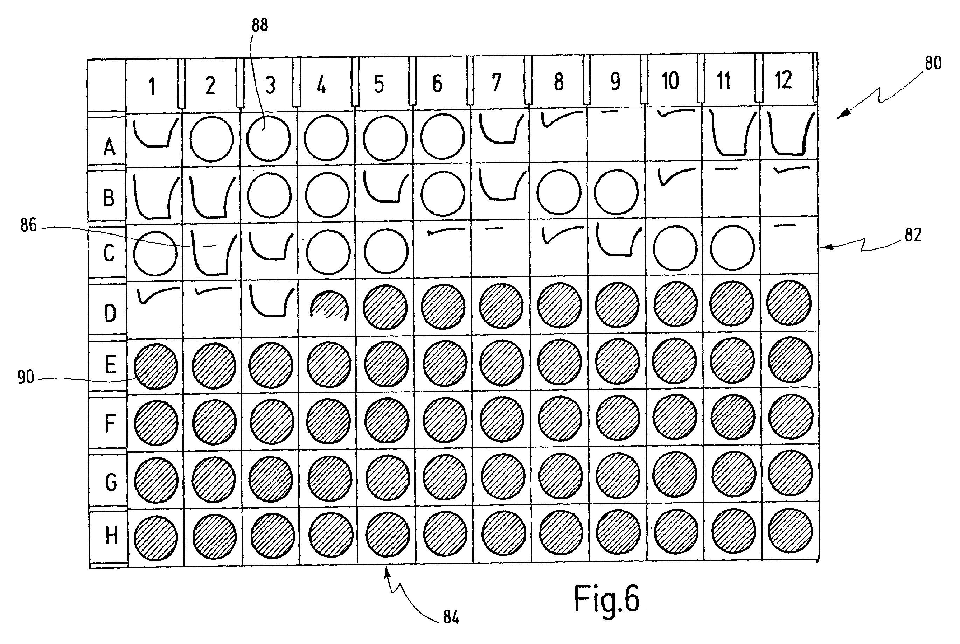

In Fig. 6 erkennt man ein zugehöriges Meßprotokoll 80. Das Meßprotokoll 80 ist in Zeilen 82 und Spalten 84 unterteilt, die der Verteilung der Wells 12 in der Well-Platte 10 entsprechen. Im dargestellten Beispiel umfaßt das Meßprotokoll 80 bei einer 96 Well-Platte Zeilen 82 mit zwölf Positionen 1 bis 12 sowie Spalten 84 mit acht Positionen A bis H.

In Fig. 6 ist ein erstes Feld mit den Koordinaten C2 mit 86, ein zweites Feld mit den Koordinaten A3 mit 88 und ein drittes Feld mit den Koordinaten E1 mit 90 bezeichnet.

Im ersten Feld 86 erkennt man einen Stromverlauf vor und während der Applikationszugabe, der bei einer erfolgreichen Messung der Oozyte 16 entspricht, die sich im Well 12 an der Koordinatenposition C2 befindet.

Das zweite Feld 88 hingegen kennzeichnet eine erfolglose Messung, die dort symbolisch mit einem Kreis angedeutet ist.

Das Meßprotokoll 80 zeigt den Zustand, bei dem gerade die Wells 12 bis hin zum Well an der Koordinatenposition D4 erfaßt worden sind. Alle darauffolgenden Wells 12 sind noch nicht vermessen, wie z.B. im dritten Feld 90 mit einem kreisförmigen Flecken angedeutet ist.

Der Benutzer der Vorrichtung kann daher durch Betrachten des Meßprotokolls 80 unmittelbar erkennen, in welchen Wells erfolgreiche Messungen durchgeführt werden konnten, in welchen Wells die Messungen erfolglos waren und welche Wells noch nicht untersucht worden sind.

Wenn für ein bestimmtes Well 12 die Messung abgeschlossen ist, wird der Meßkopf 20 in z-Richtung wieder angehoben, und es kann nun durch Umschalten des Eingangs der Förderpumpe 70 auf ein anderes Vorratsgefäß, in dem sich eine Spülflüssigkeit befindet, diese Spülflüssigkeit dem Well 12 zugeführt werden. Die Spülflüssigkeit wird nun im Durchlaufverfahren durch den Perfusateinlaß 38 zugeführt und etwas oberhalb mit dem Perfusatauslaß 40 wieder abgesaugt, bis schlußendlich die Oozyte 16 vollkommen gespült ist und alle Spuren der zuvor eingebrachten Meßsubstanz beseitigt sind.

Es kann sich dann eine weitere Messung anschließen, in der eine andere Meßsubstanz in der bereits beschriebenen Weise zugeführt wird. Die andere Meßsubstanz kann dabei die gleiche Meßsubstanz wie zuvor, jedoch in anderer Konzentration sein, es können jedoch auch völlig andere Meßsubstanzen zugeführt werden.

Es versteht sich ferner, daß die vorstehend erläuterten Meßund Spülschritte auch alle in derselben Vertikalstellung z des Meßkopfes 20 vorgenommen werden können, je nachdem, wie dies die Umstände des Einzelfalls zweckmäßig erscheinen lassen.

Mit der erfindungsgemäßen Vorrichtung können jedoch auch noch andere Funktionen außerhalb der eigentlichen Messungen durchgeführt werden:

Die in den Wells 12 befindlichen Oozyten 16 werden nämlich über längere Zeiträume nicht vermessen und daher nicht mit Testflüssigkeiten versehen, so daß die Oozyten 16 während dieser längeren Pausen Gefahr laufen, auszutrocknen. Diese Gefahr besteht beispielsweise während der typischerweise zweitägigen Inkubationszeit nach dem anfänglichen Injizieren mit cDNA/mRNA. Weitere längere Pausen können zwischen aufeinanderfolgenden Messungen entstehen, wenn nur ein Meßkopf 20 eingesetzt wird und nacheinander die 96, 384 oder gar 1.536 Wells 12 genormter Well-Platten 10 vermessen werden.

Um während dieser längeren Pausen vor den eigentlichen Messungen oder zwischen zwei Messungen ein Austrocknen der Oozyten 16 zu verhindern, kann der Meßkopf 20 in der bereits erwähnten Weise mittels des Aktuators 18 über die in Frage kommenden Wells 12 gefahren werden, damit dann über das Perfusionssystem 38/40 eine Flüssigkeit auf die Oozyten 16 gebracht werden kann, die deren Austrocknen sicher verhindert.

Bei der erfindungsgemäßen Vorrichtung wird der Meßkopf 20 herstellerseitig gefertigt. Dies bedeutet, daß alle Einzelkomponenten herstellerseitig auf dem Meßkopf 20 montiert und unter mikroskopischer Kontrolle ausgerichtet werden. Der so gefertigte Meßkopf 20 wird dann dem Benutzer übergeben. Dieser kann den Meßkopf 20 mit wenigen Handgriffen am Aktuator 18 befestigen bzw. bei Bedarf austauschen. Der Meßkopf 20 kann sofort, nach Befüllen der Ableitelektroden 30, 34 mit entsprechend leitfähigen Lösungen, auf der z-Achse montiert werden. Nach Anschließen der elektrischen Verbindungen und des Perfusionssystems kann sofort mit dem Meßvorgang begonnen werden. Diese "plug-andplay"-Eigenschaft des Meßkopfes 20 stellt eine wesentliche Erleichterung für den Anwender dar. Im Gegensatz zu herkömmlichen Vorrichtungen braucht der Anwender nämlich die Elektroden nicht selbst herzustellen, diese auszurichten, einzelne Elektroden zu chlorieren und die Referenzelektroden anzupassen. Schließlich entfällt auch die Anbringung und Positionierung der Perfusatleitungen.

Es versteht sich, daß bei der erfindungsgemäßen Vorrichtung der Meßkopf 20 mit beliebigen Kombinationen von Elektroden und Perfusatleitungen versehen werden kann. Besonders bevorzugt ist im vorliegenden Fall jedoch die Anordnung mit insgesamt sechs Elementen, wie sie in Fig. 5 im einzelnen dargestellt sind.

Der Meßkopf 20 wird zwar bevorzugt in automatischen Aktuatoren oder Robotersystemen eingesetzt, kann aber auch in herkömmlichen manuellen Systemen, insbesondere sogenannten Mikromanipulatoren eingesetzt werden.

## Patentansprüche

1. Vorrichtung zum Durchführen elektrophysiologischer Messungen an Zellen (16), mit mindestens einer Elektrode (30 - 36), mit einer ersten Perfusatleitung, die als Perfusateinlaß (38) mit einer ersten Mündung (39) zum Zuführen von Perfusat zu den Zellen (16) ausgebildet ist, und mit einer zweiten Perfusatleitung, die als Perfusatauslaß (40) mit einer zweiten Mündung (41) zum Abführen von Perfusat von den Zellen (16) ausgebildet ist, wobei die zweite Mündung (41) oberhalb der ersten Mündung (39) angeordnet ist, **dadurch gekennzeichnet, daß** die mindestens eine Elektrode (30 - 36) zum Einstechen in die Zellen (16) ausgebildet und mit den Perfusatleitungen (38, 40) in einem gemeinsamen Träger (22) eines Meßkopfes (20) integriert ist, daß der Perfusateinlaß (38) sich im wesentlichen parallel zu der mindestens einen Elektrode (30 - 36) erstreckt, und daß die erste Mündung (39) oberhalb eines unteren Endes der mindestens einen Elektrode (30 - 36) angeordnet ist.

2. Vorrichtung nach Anspruch 1, **dadurch gekennzeichnet, daß** die mindestens eine Elektrode (30 - 36) in Aussparungen des Trägers eingesetzt ist.

3. Vorrichtung nach Anspruch 1, **dadurch gekennzeichnet, daß** die mindestens eine Elektrode (30 - 36) im Träger vergossen ist.

4. Vorrichtung nach einem oder mehreren der Ansprüche 1 bis 3, **dadurch gekennzeichnet, daß** die mindestens eine Elektrode (30, 34) aus gezogenen Glasröhrchen besteht.

5. Vorrichtung nach einem oder mehreren der Ansprüche 1 bis 4, **dadurch gekennzeichnet, daß** die mindestens eine Elektrode (30, 34) einen elektrischen Widerstand zwischen 5 MΩ und 100 MΩ aufweist.

6. Vorrichtung nach einem oder mehreren der Ansprüche 1 bis 4, **dadurch gekennzeichnet, daß** die mindestens eine Elektrode (30, 34) einen elektrischen Widerstand zwischen 500 kΩ und 5 MΩ aufweist.

7. Vorrichtung nach einem oder mehreren der Ansprüche 1 bis 3, **dadurch gekennzeichnet, daß** die mindestens eine Elektrode (30 - 36) als Drahtelektrode, vorzugsweise als Silberdrahtelektrode, insbesondere als mit einer Chloridschicht versehene Silberdrahtelektrode ausgebildet ist.

8. Vorrichtung nach einem oder mehreren der Ansprüche 1 bis 7, **dadurch gekennzeichnet, daß** die mindestens eine Elektrode (30 - 36) einen geraden Abschnitt (42, 46) aufweist.

9. Vorrichtung nach einem oder mehreren der Ansprüche 1 bis 8, **dadurch gekennzeichnet, daß** mindestens eine Elektrode (30 - 36) an ihrem vorderen Ende mit einer Spitze (44, 48) versehen ist.

10. Vorrichtung nach einem oder mehreren der Ansprüche 1 bis 9, **dadurch gekennzeichnet, daß** zwei Elektroden (30 - 36) im wesentlichen symmetrisch zu einer Längsachse (24) des Trägers (22) angeordnet sind, wobei vorzugsweise die Elektroden (30, 34) an ihrem freien Ende einen Abstand (d) zwischen 50 µm und 800 µm, vorzugsweise zwischen 200 µm und 500 µm aufweisen.

11. Vorrichtung nach Anspruch 10, **dadurch gekennzeichnet, daß** mindestens eine Elektrode (30 - 36) einen geraden Abschnitt (42, 46) aufweist und daß der gerade Abschnitt (42, 46) mit einer Längsachse (24) des Trägers (22) einen spitzen Winkel (α) einschließt, der vorzugsweise zwischen 3° und 10°, insbesondere 5° beträgt.

12. Vorrichtung nach einem oder mehreren der Ansprüche 1 bis 11, **dadurch gekennzeichnet, daß** mindestens eine der Elektroden (30 - 36) als Meßelektrode (30, 34) ausgebildet ist, und daß die Meßelektrode (34) an einen vorzugsweise einstellbaren (58) Meßverstärker (56) angeschlossen ist.

13. Vorrichtung nach Anspruch 12, **dadurch gekennzeichnet, daß** mindestens eine Meßelektrode (30) an eine vorzugsweise einstellbare (52) Stromquelle (50) angeschlossen ist.

14. Vorrichtung nach einem oder mehreren der Ansprüche 1 bis 13, **dadurch gekennzeichnet, daß** mindestens eine der Elektroden (30 - 36) als Referenzelektrode (32, 36) ausgebildet ist, die vorzugsweise an eine Masse (54) angeschlossen ist.

15. Vorrichtung nach Anspruch 14, **dadurch gekennzeichnet, daß** zwei Meßelektroden (30, 34) und zwei Referenzelektroden (32, 36) vorgesehen sind.

16. Vorrichtung nach einem oder mehreren der Ansprüche 12 bis 15, **dadurch gekennzeichnet, daß** mindestens zwei Meßelektroden (30, 34) in einer ersten gemeinsamen Ebene (35) angeordnet sind, wobei vorzugsweise mindestens zwei Referenzelektroden (32, 36) in einer zweiten gemeinsamen Ebene (37) angeordnet sind, und insbesondere die erste und die zweite Ebene (35, 37) parallel zueinander verlaufen.

17. Vorrichtung nach einem oder mehreren der Ansprüche 11 bis 16, **dadurch gekennzeichnet**, der Perfusateinlaß (38) im wesentlichen auf der Symmetrieachse zwischen den Meßelektroden (30, 34) angeordnet ist.

18. Vorrichtung nach Anspruch 17, **dadurch gekennzeichnet, daß** der Perfusateinlaß (38) an eine vorzugsweise einstellbare (72) Förderpumpe (70) angeschlossen ist.

19. Vorrichtung nach Anspruch 17 oder 18, **dadurch gekennzeichnet, daß** der Perfusateinlaß (38) über ein steuerbares Ventilsystem an eine Mehrzahl von Vorratsbehältern anschließbar ist, wobei vorzugsweise mindestens ein Vorratsbehälter eine Testflüssigkeit enthält und weiter vorzugsweise mindestens ein Vorratsbehälter eine Spülflüssigkeit enthält.

20. Vorrichtung nach Anspruch 19, **dadurch gekennzeichnet, daß** die Vorratsbehälter oberhalb des Perfusateinlasses (38) angeordnet sind.

21. Vorrichtung nach einem oder mehreren der Ansprüche 1 bis 20, **dadurch gekennzeichnet, daß** die erste und die zweite Mündung (39, 41) entgegengesetzt gerichtet sind.

22. Vorrichtung nach einem oder mehreren der Ansprüche 1 bis 21, **dadurch gekennzeichnet, daß** der Perfusatauslaß (40) an eine vorzugsweise einstellbare (76) Saugpumpe (74) angeschlossen ist.

23. Vorrichtung nach Anspruch 16, **dadurch gekennzeichnet, daß** in Blickrichtung auf die erste Ebene (35) der Perfusateinlaß (38) vor der ersten Ebene (35) und der Perfusatauslaß (40) hinter der zweiten Ebene (37) angeordnet ist.

24. Vorrichtung nach einem oder mehreren der Ansprüche 1 bis 23, **dadurch gekennzeichnet, daß** mindestens ein Meßkopf (20) an einem Aktuator (18) angeordnet ist, und daß der Aktuator (18) entlang eines Koordinatensystems (14) oberhalb einer Aufnahme für die Zellen (16) verfahrbar ist.

25. Vorrichtung nach Anspruch 24, **dadurch gekennzeichnet, daß** der Aktuator (18) eine Mehrzahl von Meßköpfen (20, 20a, 20b) trägt, wobei vorzugsweise die Meßköpfe (20) relativ zum Aktuator (18) mindestens entlang der auf die Zelle (16) gerichteten Achse (z) individuell verfahrbar sind.

26. Vorrichtung nach Anspruch 24 oder 25, **dadurch gekennzeichnet, daß** der Meßkopf (20) steckbar oder schraubbar am Aktuator (18) befestigt ist.

27. Vorrichtung nach einem oder mehreren der Ansprüche 1 bis 26, **dadurch gekennzeichnet, daß** Mittel zum Injizieren von cDNA und/oder mRNA in die Zelle (16) vorgesehen sind, wobei vorzugsweise die Mittel am Aktuator (18) angeordnet sind.

28. Vorrichtung nach einem oder mehreren der Ansprüche 24 bis 27, **dadurch gekennzeichnet, daß** die Aufnahme für die Zellen (16) als standardisierte Multi-Well-Platte (10) ausgebildet ist.

29. Vorrichtung nach Anspruch 28, **dadurch gekennzeichnet, daß** die einzelnen Aufnahmen (12) in der Platte (10) mit einer lesbaren Codierung (13) versehen sind und daß der Aktuator (18) Mittel zum Lesen der Codierung umfaßt, wobei vorzugsweise die Codierung eine Bar-Codierung ist und die Mittel ein Bar-Code-Lesekopf (19) sind.
